# EUROPÄISCHE PATENTANMELDUNG

(11) **EP 2 145 540 A1**
(43) Veröffentlichungstag der Anmeldung: **20.01.2010**
(21) Anmeldenummer: 09169505.6
(22) Anmeldetag: 05.11.1998
(51) Int. Cl.: A01N 43/80, C09C 3/04, C07D 275/02

(54) **Isothiazolcarbonsäureamide und deren Verwendung zum Schutz von Pflanzen**

(30) Priorität: 05.11.1997 DE 19750012
(62) Teilanmeldung aus: 02017799.4
(71) Anmelder: Bayer CropScience AG, 40789 Monheim (DE)
(72) Erfinder: Aßmann, Lutz, Dr., 40764 Langenfeld (DE); Kuhnt, Dietmar, Dr., 51399 Burscheid (DE); Elbe, Hans-Ludwig, Dr., 42329 Wuppertal (DE); Erdelen, Christoph, Dr., 42799 Leichlingen (DE); Dutzmann, Stefan, Dr., 40764 Langenfeld (DE); Hänßler, Gerd, Dr., 51381 Leverkusen (DE); Stenzel, Klaus, Dr., 40595 Düsseldorf (DE); Mauler-Machnik, Astrid, Dr., 42799 Leichlingen (DE); Kitagawa, Yoshinori, Dr., Tichigi-Ken 321-4305 (JP); Kawada, Haruko, 307-0007 Yuki-ski, Ibaraki (JP); Sakuma, Haruhiko, 323-0829 Tochigi (JP)

(57) **Zusammenfassung**

Neue Isothiazolcarbonsäureamide der Formel 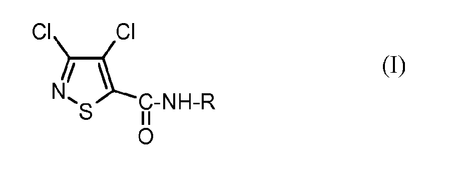 in welcher
R
die in der Beschreibung angegebenen Bedeutungen hat,

mehrere Verfahren zur Herstellung der neuen Stoffe und deren Verwendung zum Schutz von Pflanzen gegen Befall durch unerwünschte Mikroorganismen und tierische Schädlinge.

## Beschreibung

Die vorliegende Erfindung betrifft neue Isothiazolcarbonsäureamide, mehrere Verfahren zu deren Herstellung und deren Verwendung zum Schutz von Pflanzen gegen Befall durch unerwünschte Mikroorganismen und tierische Schädlinge.

Es ist bereits bekannt geworden, dass zahlreiche Isothiazolcarbonsäure-Derivate fungizide Eigenschaften besitzen (vgl. US-A 5 240 951 und JP-A 06-009 313). So lassen sich z.B. 3,4-Dichlor-isothiazol-5-carbonsäure-ethylamid und 3,4-Dichlor-isothiazol-5-carbonsäure-(4-chlor-anilid) zur Bekämpfung von Pilzen einsetzen. Die Wirksamkeit dieser Stoffe ist gut, lässt aber bei niedrigen Aufwandmengen in manchen Fällen zu wünschen übrig.

Es wurden nun neue Isothiazolcarbonsäureamide der Formel 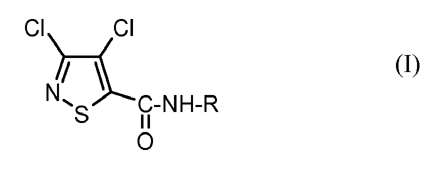 in welcher
- R: für einen Rest der Formel 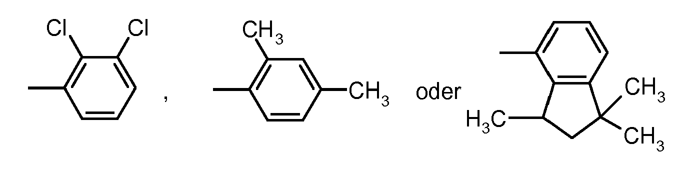 steht, oder
- R: für einen Rest der Formel 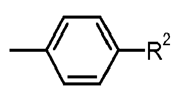 steht, worin
R² für -C(CH₃)₃, 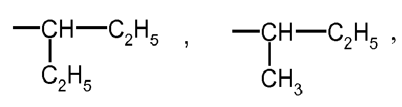 Cycloalkyl mit 3 bis 7 Kohlenstoffatomen oder für -CH₂-S-R³ steht, wobei
R³ für Alkyl mit 1 bis 6 Kohlenstoffatomen oder für gegebenenfalls durch Halogen und/oder Alkyl mit 1 bis 6 Kohlenstoffatomen substituiertes Phenyl steht, oder
- R: für einen Rest der Formel 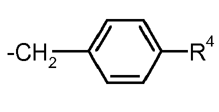 steht, worin
R⁴ für Wasserstoff oder N,N-Dialkylaminomethyl mit 1 bis 4 Kohlenstoffatomen in jedem Alkylteil steht, oder
- R: für einen Rest der Formel 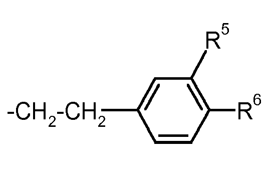 steht, worin
R⁵ für Wasserstoff oder Alkoxy mit 1 bis 4 Kohlenstoffatomen steht und
R⁶ für Alkoxy mit 1 bis 4 Kohlenstoffatomen, Alkyl mit 1 bis 6 Kohlenstoffatomen, gegebenenfalls durch Halogen substituiertes Phenyl oder für gegebenenfalls durch Halogen substituiertes Phenoxy steht,
oder
R⁵ für gegebenenfalls durch Halogen substituiertes Phenoxy steht und
R⁶ für Wasserstoff steht, oder
- R: für einen Rest der Formel 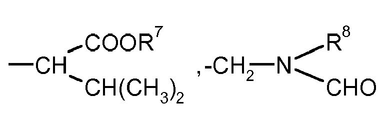 oder -CH₂-CH₂-O-R⁹ steht, worin
R⁷ für Alkyl mit 1 bis 4 Kohlenstoffatomen steht,
R⁸ für Alkyl mit 1 bis 4 Kohlenstoffatomen steht und
R⁹ für Wasserstoff oder einen Rest der Formel 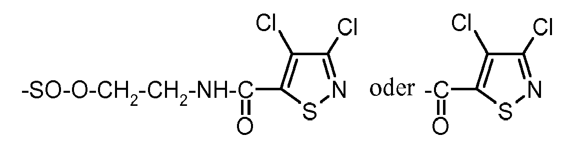 steht,
oder
- R: für einen Rest der Formel 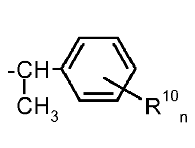 steht, worin
R¹⁰ für Halogen, Alkyl mit 1 bis 4 Kohlenstoffatomen oder Alkoxy mit 1 bis 4 Kohlenstoffatomen steht und
n für ganze Zahlen von 0 bis 3 steht,
gefunden.

Weiterhin wurde gefunden, daß sich Isothiazolcarbonsäureamide der Formel (I) herstellen lassen, indem man
a) 3,4-Dichlor-isothiazol-5-carbonsäurechlorid der Formel 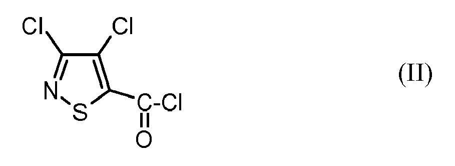 mit Aminen der Formel

   H₂N-R (III)

   in welcher
   - R: die oben angegebenen Bedeutungen hat,
   gegebenenfalls in Gegenwart eines Säurebindemittels und gegebenenfalls in Gegenwart eines Verdünnungsmittels umsetzt,
   oder
b) 3,4-Dichlor-isothiazol-5-carbonsäureamid der Formel 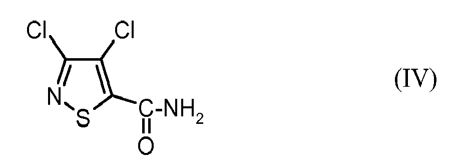 mit Hydroxyverbindungen der Formel

   HO-CH₂-X (V)

   in welcher
   - X: für einen Rest der Formel 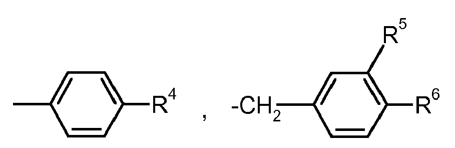 oder 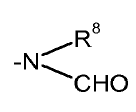 steht, worin
   R⁴, R⁵, R⁶ und R⁸ die oben angegebenen Bedeutungen haben,
   in Gegenwart eines Verdünnungsmittels und in Gegenwart eines wasserabspaltenden Mittels umsetzt.

Schließlich wurde gefunden, dass die Isothiazolcarbonsäureamide der Formel (I) sehr gut zum Schutz von Pflanzen gegen Befall durch unerwünschte Mikroorganismen verwendbar sind. Die erfindungsgemäßen Stoffe eignen sich sowohl zur Mobilisierung pflanzeneigener Abwehrkräfte gegen Befall durch unerwünschte Mikroorganismen als auch als Mikrobizide zur direkten Bekämpfung der Mikroorganismen. Außerdem zeigen die erfindungsgemäßen Stoffe auch eine Wirkung gegen tierische Pflanzenschädlinge.

Überraschenderweise besitzen die erfindungsgemäßen Stoffe eine bessere mikrobizide Wirksamkeit als 3,4-Dichlor-isothiazol-5-carbonsäure-ethylamid und 3,4-Dichlor-iso-thiazol-5-carbonsäure-(4-chlor-anilid), welches konstitutionell ähnliche, vorbekannte Wirkstoffe gleicher Wirkungsrichtung sind.

Die erfindungsgemäßen Isothiazolcarbonsäureamide sind durch die Formel (I) allgemein definiert. Bevorzugt sind Verbindungen der Formel (I), in denen
- R: für einen Rest der Formel 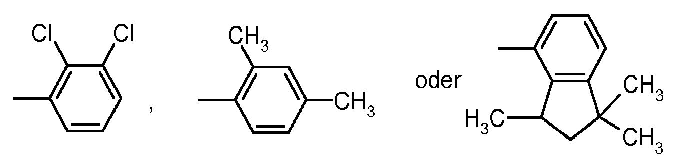 steht,
oder
- R: für einen Rest der Formel 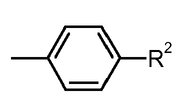 steht, worin
R² für -C(CH₃)₃, 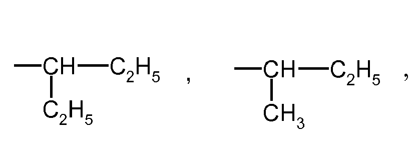 Cyclopentyl, Cyclohexyl, Cycloheptyl oder für -CH₂-S-R³ steht, wobei
R³ für Alkyl mit 1 bis 5 Kohlenstoffatomen oder für gegebenenfalls einfach bis dreifach, gleichartig oder verschieden durch Fluor, Chlor, Brom und/oder Alkyl mit 1 bis 4 Kohlenstoffatomen substituiertes Phenyl steht, oder
- R: für einen Rest der Formel 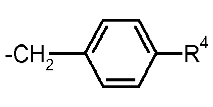 steht, worin
R⁴ für Wasserstoff oder N,N-Dialkyl-aminomethyl mit 1 oder 2 Kohlenstoffatomen in jedem Alkylteil steht, oder
- R: für einen Rest der Formel 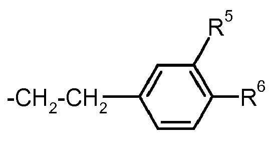 steht, worin
R⁵ für Wasserstoff oder Alkoxy mit 1 oder 2 Kohlenstoffatomen steht und
R⁶ für Alkoxy mit 1 oder 2 Kohlenstoffatomen, Alkyl mit 1 bis 4 Kohlenstoffatomen, gegebenenfalls einfach bis dreifach, gleichartig oder verschieden durch Fluor, Chlor und/oder Brom substituiertes Phenyl oder für gegebenenfalls einfach bis dreifach, gleichartig oder verschieden durch Fluor, Chlor und/oder Brom substituiertes Phenoxy steht,
oder
R⁵ für gegebenenfalls einfach bis dreifach, gleichartig oder verschieden durch Fluor, Chlor und/oder Brom substituiertes Phenoxy steht und
R⁶ für Wasserstoff steht, oder
- R: für einen Rest der Formel 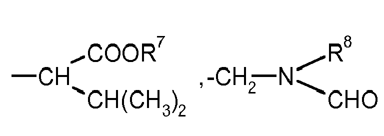 oder -CH₂-CH₂-O-R⁹ steht, worin
R⁷ für Methyl oder Ethyl steht,
R⁸ für Methyl oder Ethyl steht und
R⁹ für Wasserstoff oder einen Rest der Formel 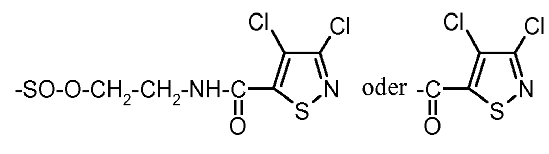 steht, oder
- R: für einen Rest der Formel 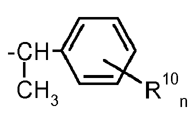 steht, worin
R¹⁰ für Fluor, Chlor, Brom, Methyl, Ethyl, Methoxy oder Ethoxy steht und
n für ganze Zahlen von 0 bis 3 steht, wobei R¹⁰ für gleiche oder verschiedene Reste steht, wenn n für 2 oder 3 steht.

Die zuvor angegebenen Substituenten-Definitionen können untereinander kombiniert werden. Außerdem können auch einzelne Definitionen entfallen.

Verwendet man 3,4-Dichlor-isothiazol-5-carbonsäureamid und N-Formyl-N-hydroxymethyl-methylamin als Ausgangsstoffe, so kann der Verlauf des erfindungsgemäßen Verfahrens (b) durch das folgende Formelschema veranschaulicht werden.

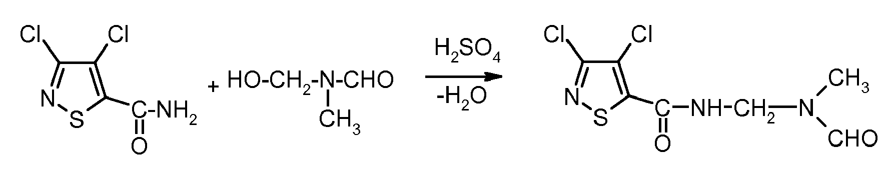

Das bei der Durchführung des erfindungsgemäßen Verfahrens (a) als Ausgangssubstanz benötigte 3,4-Dichlor-isothiazol-5-carbonsäure-chlorid der Formel (II) ist bekannt (vgl. US-A-5 240 951).

Die weiterhin bei der Durchführung des erfindungsgemäßen Verfahrens (a) als Reaktionskomponenten benötigten Amine sind durch die Formel (III) allgemein definiert. In dieser Formel hat R vorzugsweise diejenigen Bedeutungen, die bereits im Zusammenhang mit der Beschreibung der erfindungsgemäßen Stoffe der Formel (I) als bevorzugt für diesen Rest genannt wurden.

Die Amine der Formel (III) sind bekannt oder lassen sich nach bekannten Methoden herstellen.

Als Säurebindemittel kommen bei der Durchführung des erfindungsgemäßen Verfahrens (a) alle üblichen anorganischen und organischen Basen in Frage. Vorzugsweise verwendbar sind Erdalkalimetall- oder Alkalimetallhydride, -hydroxide, -amide, -alkoholate, -acetate, -carbonate oder -hydrogencarbonate, wie Natriumhydrid, Natriumamid, Natriummethylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat, ferner Ammoniumhydroxid, Ammoniumacetat oder Ammoniumcarbonat, oder tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (a) kommen alle inerten organischen Lösungsmittel in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Die Reaktionstemperaturen können bei der Durchführung des erfindungsgemäßen Verfahrens (a) innerhalb eines größeren Bereiches variiert werden. Im Allgemeinen arbeitet man bei Temperaturen zwischen -10°C und +150°C, vorzugsweise zwischen 0°C und 100°C.

Bei der Durchführung des erfindungsgemäßen Verfahrens (a) arbeitet man im Allgemeinen unter Atmosphärendruck. Es ist aber auch möglich, unter erhöhtem oder, sofern keine flüchtigen Komponenten an der Umsetzung beteiligt sind, unter vermindertem Druck zu arbeiten.

Bei der Durchführung des erfindungsgemäßen Verfahrens (a) setzt man auf 1 Mol an 3,4-Dichlor-isothiazol-5-carbonsäure-chlorid der Formel (II) im Allgemeinen 1 bis 5 Mol, vorzugsweise 1 bis 2 Mol an Amin der Formel (III) sowie eine äquivalente Menge oder einen Überschuss an Säurebindemittel ein. Die Aufarbeitung erfolgt nach üblichen Methoden. Im Allgemeinen verfährt man in der Weise, dass man das Reaktionsgemisch nach beendeter Umsetzung einengt, den verbleibenden Rückstand mit Wasser und einem mit Wasser wenig mischbaren organischen Lösungsmittel versetzt, die organische Phase abtrennt, wäscht, trocknet und einengt. Das verbleibende Produkt kann nach üblichen Methoden von eventuell enthaltenen Verunreinigungen befreit werden.

Das bei der Durchführung des erfindungsgemäßen Verfahrens (b) als Ausgangssubstanz benötigte 3,4-Dichlor-isothiazol-5-carbonsäureamid der Formel (IV) ist bekannt (vgl. US-A-5 240 951).

Die weiterhin bei der Durchführung des erfindungsgemäßen Verfahrens (b) als Reaktionskomponenten benötigten Hydroxyverbindungen sind durch die Formel (V) allgemein definiert. In dieser Formel steht
- X: vorzugsweise für einen Rest der Formel 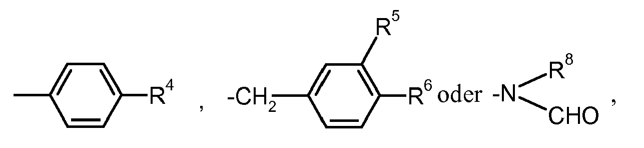 worin
R⁴ für Wasserstoff oder N,N-Dialkyl-aminomethyl mit 1 oder 2 Kohlenstoffatomen in jedem Alkylteil steht,
R⁵ für Wasserstoff oder Alkoxy mit 1 oder 2 Kohlenstoffatomen steht,
R⁶ für Alkoxy mit 1 oder 2 Kohlenstoffatomen, Alkyl mit 1 bis 4 Kohlenstoffatomen, gegebenenfalls einfach bis dreifach, gleichartig oder verschieden durch Fluor, Chlor und/oder Brom substituiertes Phenyl oder für gegebenenfalls einfach bis dreifach, gleichartig oder verschieden durch Fluor, Chlor und/oder Brom substituiertes Phenoxy steht,
oder
R⁵ für gegebenenfalls einfach bis dreifach, gleichartig oder verschieden durch Fluor, Chlor und/oder Brom substituiertes Phenoxy steht und
R⁶ für Wasserstoff steht und
R⁸ für Methyl oder Ethyl steht.

Die Hydroxyverbindungen der Formel (V) sind bekannt oder lassen sich nach bekannten Methoden herstellen.

Als Verdünnungsmittel kommen bei der Durchführung des erfindungsgemäßen Verfahrens (b) alle für derartige Umsetzungen üblichen, inerten organischen Solventien in Frage. Vorzugsweise verwendbar ist Eisessig.

Als wasserabspaltende Mittel kommen bei der Durchführung des erfindungsgemäßen Verfahrens (b) alle üblichen Reagenzien in Betracht, die zur Dehydratation befähigt sind. Vorzugsweise verwendbar sind Säuren, wie Schwefelsäure oder p-Toluolsulfonsäure, und auch Trockenmittel, wie wasserfreies Kieselgel.

Die Reaktionstemperaturen können auch bei der Durchführung des erfindungsgemäßen Verfahrens (b) innerhalb eines größeren Bereichs variiert werden. Im Allgemeinen arbeitet man bei Temperaturen zwischen 0°C und 150°C, vorzugsweise zwischen 10°C und 130°C.

Auch bei der Durchführung des erfindungsgemäßen Verfahrens (b) arbeitet man im Allgemeinen unter Atmosphärendruck. Es ist aber auch möglich, unter erhöhtem oder vermindertem Druck zu arbeiten.

Bei der Durchführung des erfindungsgemäßen Verfahrens (b) setzt man auf 1 Mol an 3,4-Dichlor-isothiazol-5-carbonsäureamid der Formel (IV) im Allgemeinen 1 bis 2 Mol, vorzugsweise 1 bis 1,5 Mol an Hydroxyverbindung der Formel (V) sowie 2 bis 6 Mol an wasserabspaltendem Mittel ein. Die Aufarbeitung erfolgt nach üblichen Methoden. Im Allgemeinen verfährt man in der Weise, dass man das Reaktionsgemisch mit Wasser versetzt, dann mit einem mit Wasser wenig mischbaren organischen Solvens extrahiert, die vereinigten organischen Phasen trocknet und unter vermindertem Druck einengt. Das verbleibende Produkt kann nach üblichen Methoden von eventuell noch vorhandenen Verunreinigungen befreit werden.

Die erfindungsgemäßen Wirkstoffe weisen eine starke pflanzenstärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die erfindungsgemäßen Wirkstoffe weisen neben der pflanzenstärkenden (resistenzinduzierenden) Wirkung auch eine starke mikrobizide Wirkung auf und werden auch zur direkten Bekämpfung von unerwünschten Mikroorganismen praktisch eingesetzt. Die Wirkstoffe sind für den Gebrauch als Pflanzenschutzmittel, insbesondere als Fungizide geeignet.

Zu den unerwünschten Mikroorganismen im Pflanzenschutz gehören Pilze aus den Klassen Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, oder von Krankheiten im Wein-, Obst- und Gemüseanbau, wie beispielsweise gegen Plasmopara- oder Venturia-Arten, oder von Reiskrankheiten, wie beispielsweise gegen Pyricularia-Arten einsetzen. Mit gutem Erfolg lassen sich mit den erfindungsgemäßen Wirkstoffen auch weitere Pflanzenkrankheiten, wie beispielsweise Septoria-, Cochliobolus-, Pyrenophora- und Pseudocercosporella-Arten bekämpfen, wobei Drechslera teres speziell genannt sei.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäß verwendbaren Wirkstoffe eignen sich bei guter Pflanzenverträglichkeit und günstiger Warmblütertoxizität auch zur Bekämpfung von tierischen Schädlingen, insbesondere von Insekten, Spinnentieren und Nematoden, die in der Landwirtschaft, in Forsten, im Gartenbau, im Vorrats- und Materialschutz sowie auf dem Hygienesektor bzw. im veterinärmedizinischen Bereich vorkommen. Die Stoffe sind gegen normal sensible und resistente Arten sowie gegen Schädlinge in allen oder einzelnen Entwicklungsstadien wirksam. Zu den oben erwähnten tierischen Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp.

Die erfindungsgemäß verwendbaren Stoffe lassen sich mit besonders gutem Erfolg zur Bekämpfung von pflanzenschädigenden Milben, wie gegen die Bohnenspinnmilbe (Tetranychus urticae), oder zur Bekämpfung von pflanzenschädigenden Insekten, wie gegen die Raupen der Kohlschabe (Plutella maculipennis), die Larven des Meerettichblattkäfers (Phaedon cochleariae), sowie der grünen Reiszikade (Nephotettix cincticeps), einsetzen.

Die erfindungsgemäßen Stoffe besitzen auch eine herbizide Wirksamkeit.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α -(1,1-Dimethylethyl)-β-(2-phenoxyethyl)-1H-1,2,4-triazo1-1-ethanol,
   α -(2,4-Dichlorphenyl)-β-fluor-b-propyl-1H-1,2,4-triazol-1-ethanol,
   α -(2,4-Dichlorphenyl)-β-methoxy-a-methyl-1H-1,2,4-triazol-1-ethanol,
   α -(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-α-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester,
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholin-hydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on.

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos,
   N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lamda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
   Quinalphos,
   Salithion, Sebufos, Silafluofen, Sulfotep, Sulprofos,
   Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe zur Bekämpfung von Mikroorganismen können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10.000 g/ha, vorzugsweise zwischen 10 und 1.000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Auch beim Einsatz gegen tierische Schädlinge können die erfindungsgemäßen Stoffe in handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Die Herstellung und die Verwendung der erfindungsgemäßen Wirkstoffe wird durch die folgenden Beispiele veranschaulicht.

### Herstellungsbeispiele

### Beispiel 1

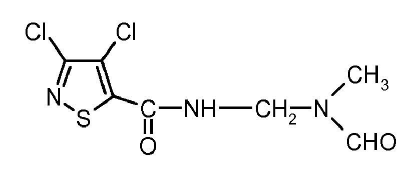

In ein Gemisch aus 32,3 g (0,164 Mol) 3,4-Dichlor-isothiazol-5-carbonsäureamid und 245 ml Eisessig werden unter Rühren bei Raumtemperatur 15 g (0,164 Mol) N-Formyl-N-hydroxymethyl-methylamin gegeben. Danach tropft man unter Rühren bei Raumtemperatur 37 g (0,362 Mol) konzentrierte Schwefelsäure hinzu, wobei das Reaktionsgemisch mit Eis gekühlt wird. Anschließend wird das Reaktionsgemisch 29 Stunden bei Raumtemperatur gerührt und dann unter Eiskühlung mit 400 ml Wasser versetzt. Man extrahiert das entstehende Gemisch viermal mit je 200 ml Methylenchlorid, trocknet die vereinigten organischen Phasen über Natriumsulfat und engt dann unter vermindertem Druck ein. Der verbleibende ölige Rückstand wird mit Essigsäureethylester an Kieselgel chromatographiert. Nach dem Einengen des Eluates erhält man 20,7 g (42,6 % der Theorie) an 3,4-Dichlor-isothiazol-5-carbonsäure-(N-formyl-N-methyl-amino)-methylamid in Form einer kristallinen Festsubstanz vom Schmelzpunkt 87 bis 88°C.

### Herstellung der Ausgangssubstanz:

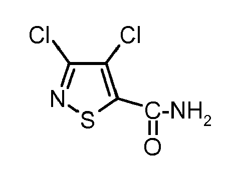

In 82 g (1,2 Mol) konzentrierten Ammoniak werden bei 0 bis 10°C innerhalb von 30 Minuten unter Rühren 50,1 g (0,2 Mol) 3,4-Dichlor-isothiazol-5-carbonsäurechlorid eingetropft. Nach beendeter Zugabe fügt man erneut 41 g (0,6 Mol) konzentrierten Ammoniak hinzu und verdünnt mit 70 ml Wasser. Man lässt auf Raumtemperatur erwärmen und rührt 45 Minuten bei dieser Temperatur. Der entstandene Niederschlag wird abgesaugt, nacheinander mit Wasser und Petrolether gewaschen und getrocknet. Man erhält auf diese Weise 32,3 g (81,8 % der Theorie) an 3,4-Dichlorisothiazol-5-carbonsäureamid in Form einer Festsubstanz vom Schmelzpunkt 156 bis 158°C.

Nach den zuvor angegebenen Methoden werden auch die in der folgenden Tabelle aufgeführten Stoffe der Formel (I) hergestellt.

**Tabelle 1**

| | | |
|---|---|---|
| 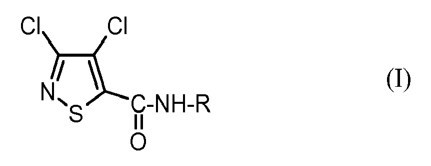 | | |

| Beispiel-Nr. | R | Physikalische Konstanten |
|---|---|---|
| 2 | 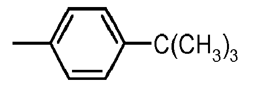 | Fp = 148-149°C |
| 3 | 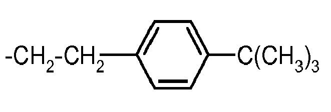 | log P = 4,81*); λ = 218, 244 u. 266 nm**) |
| 4 | 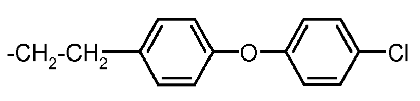 | log P = 4,86; λ = 234 u. 270 nm |
| 5 | 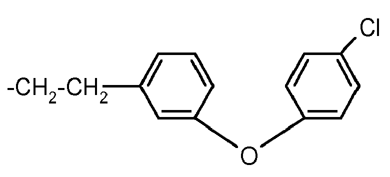 | log p = 4,79; λ = 232 u. 270 nm |
| 6 | 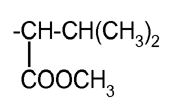 | log P=3,15; λ = 242 u. 266 nm |
| 7 | 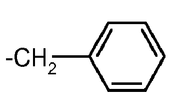 | Fp = 91-92°C |
| 8 | 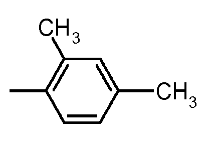 | Fp = 171-172°C |
| 9 | 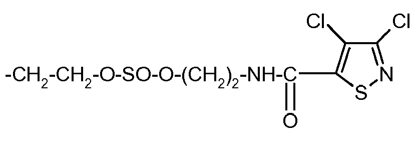 | Fp = 70-75°C |
| 10 | -CH₂-CH₂-OH | Fp = 115-117°C |
| 11 | 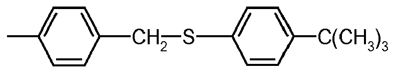 | Fp = 126-128°C |
| 12 | 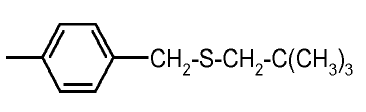 | Fp = 91-92°C |
| 13 | 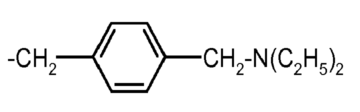 | log P = 1,39; λ = 219 u. 243 nm |
| 14 | 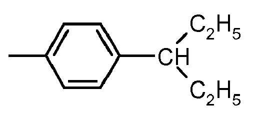 | Fp = 98-99°C |
| 15 | 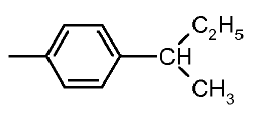 | Fp = 83-85°C |
| 16 | 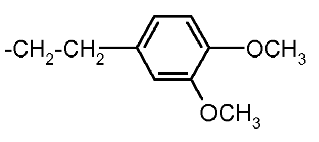 | Fp = 81-83°C |
| 17 | 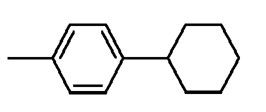 | Fp = 162-163°C |
| 18 | 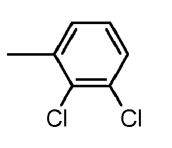 | Fp = 196°C |
| 19 | 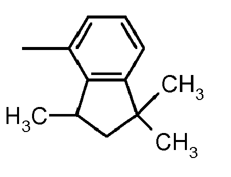 | Fp = 129-130°C |
| 20 | 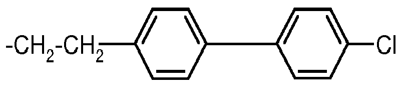 | Fp = 135-136°C |
| 21 | 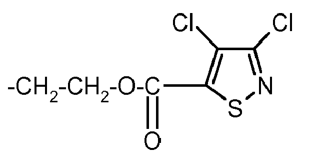 | Fp = 88-89°C |
| 22 | 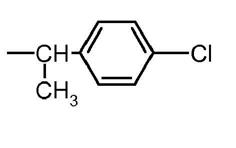 | Fp = 89-90°C |

| | | |
|---|---|---|
| *) Die Bestimmung der log P-Werte erfolgte gemäß EEC-Directive 79/831 Annex V. A8 durch HPLC (Gradientenmethode, Acetonitril/0,1 % wässrige Phosphorsäure). **) Die λ-Werte bezeichnen Maxima im UV-Spektrum. | | |

### Verwendungsbeispiele

### Beispiel A

### Pyricularia-Test (Reis) / Resistenzinduktion

| | | |
|---|---|---|
| Lösungsmittel : | 2,5 | Gewichtsteile Aceton |
| Emulgator: | 0,06 | Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf resistenzinduzierende Wirksamkeit bespritzt man junge Reispflanzen mit der Wirkstoffzubereitung in der angegebenen Aufwandmenge. 5 Tage nach der Behandlung werden die Pflanzen mit einer wässrigen Sporensuspension von Pyricularia oryzae inokuliert. Anschließend werden die Pflanzen in einem Gewächshaus bei 100 % relativer Luftfeuchtigkeit und einer Temperatur von 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle A**

| **Pyricularia-Test (Reis) / Resistenzinduktion** | | |
|---|---|---|
| Wirkstoff | Aufwandmenge an Wirkstoff in g/ha | Wirkungsgrad in % |
| Erfindungsgemäß: | | |
| 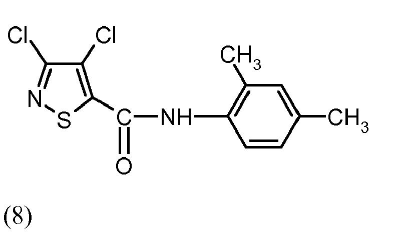 | 750 | 90 |
| 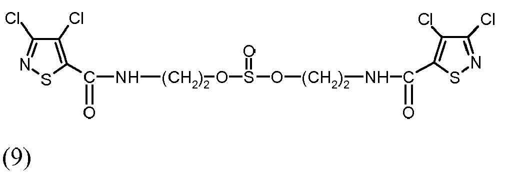 | 750 | 90 |
| 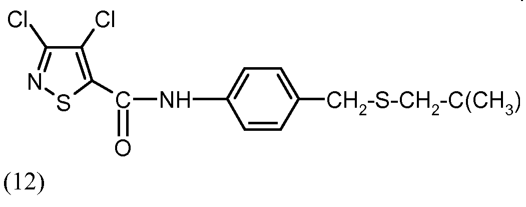 | 750 | 90 |
| 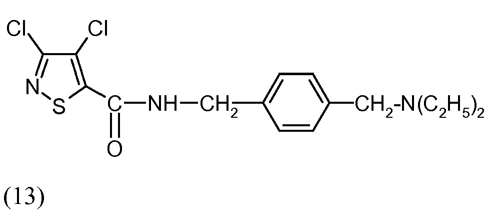 | 750 | 100 |
| 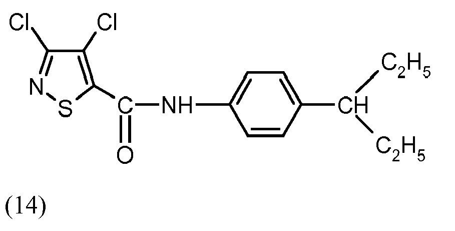 | 750 | 90 |
| 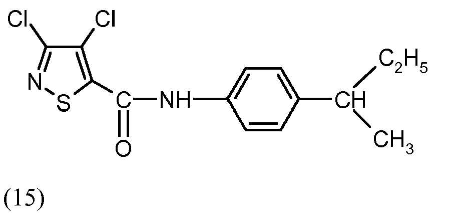 | 750 | 90 |

### Beispiel B

### Phaedon-Larven-Test

| | | |
|---|---|---|
| Lösungsmittel : | 7 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach 7 Tagen wird der Abtötungsgrad bestimmt und in Prozent ausgedrückt. Dabei bedeutet ein Wirkungsgrad von 100 %, dass alle Käferlarven abgetötet wurden, während ein Wirkungsgrad von 0 % bedeutet, dass keine Käferlarven abgetötet wurden.

Wirkstoffe, Wirkstoffkonzentrationen und Versuchsergebnisse gehen aus der folgenden Tabelle hervor.

**Tabelle B**

| **Phaedon-Larven-Test / pflanzenschädigende Insekten** | | |
|---|---|---|
| Wirkstoff | Wirkstoffkonzentration in Gew.-% | Abtötungsgrad in % nach 7^{d} |
| 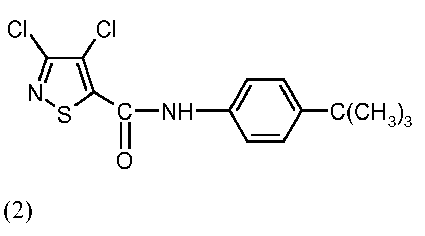 | 0,1 | 100 |

### Beispiel C

### Plutella-Test

| | | |
|---|---|---|
| Lösungsmittel: | 7 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach 7 Tagen wird der Abtötungsgrad bestimmt und in Prozent ausgedrückt. Dabei bedeutet ein Wirkungsgrad von 100 %, dass alle Raupen abgetötet wurden, während ein Wirkungsgrad von 0 % bedeutet, dass keine Raupen abgetötet wurden.

Wirkstoffe, Wirkstoffkonzentrationen und Versuchsergebnisse gehen aus der folgenden Tabelle hervor.

**Tabelle C**

| **Plutella-Test / pflanzenschädigende Insekten** | | |
|---|---|---|
| Wirkstoff | Wirkstoffkonzentration in Gew.-% | Abtötungsgrad in % nach 7^{d} |
| 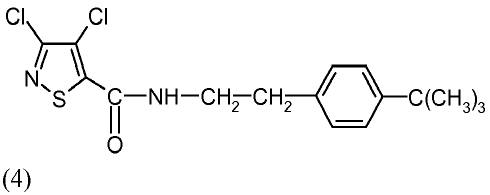 | 0,1 | 100 |
| 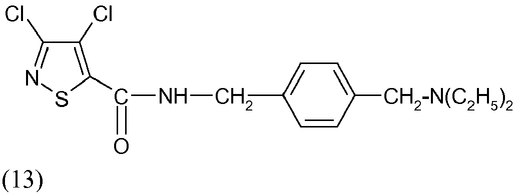 | 0,1 | 100 |
| 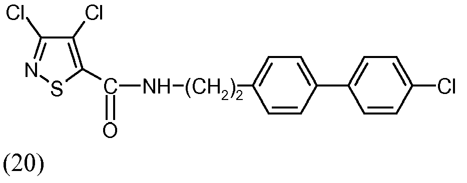 | 0,1 | 100 |

### Beispiel D

### Venturia-Test (Apfel) / protektiv

| | | |
|---|---|---|
| Lösungsmittel: | 47 | Gewichtsteile Aceton |
| Emulgator: | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle D**

| **Venturia-Test (Apfel) / protektiv** | | |
|---|---|---|
| Wirkstoff | Aufwandmenge an Wirkstoff in g/ha | Wirkungsgrad in % |
| Erfindungsgemäß: | | |
| 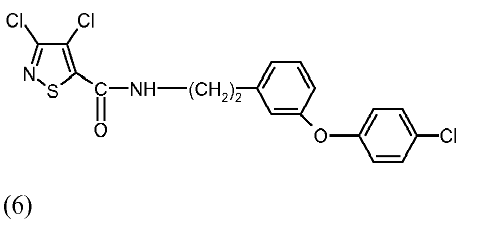 | 100 | 96 |

### Beispiel E

### Leptosphaeria nodorum-Test (Weizen) / protektiv

| | | |
|---|---|---|
| Lösungsmittel: | 10 | Gewichtsteile N-Methyl-pyrrolidon |
| Emulgator: | 0,6 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von 80 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle E**

| **Leptosphaeria nodorum-Test (Weizen) / protektiv** | | |
|---|---|---|
| Wirkstoff | Aufwandmenge an Wirkstoff in g/ha | Wirkungsgrad in % |
| Erfindungsgemäß: | | |
| 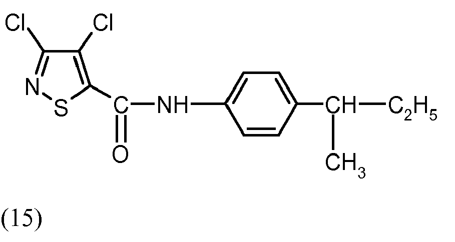 | 250 | 81 |

## Patentansprüche

1. Isothiazolcarbonsäureamide der Formel 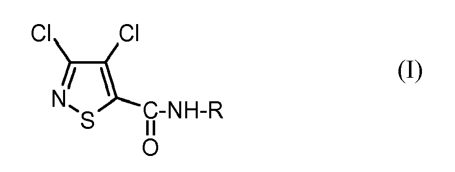 in welcher
R für einen Rest der Formel 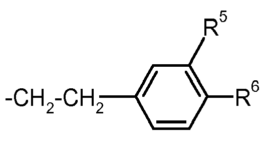 steht, worin
R⁵ für Wasserstoff oder Alkoxy mit 1 bis 4 Kohlenstoffatomen steht und
R⁶ für Alkoxy mit 1 bis 4 Kohlenstoffatomen, Alkyl mit 1 bis 6 Kohlenstoffatomen, gegebenenfalls durch Halogen substituiertes Phenyl oder für gegebenenfalls durch Halogen substituiertes Phenoxy steht,
oder
R⁵ für gegebenenfalls durch Halogen substituiertes Phenoxy steht und
R⁶ für Wasserstoff steht,
oder
R für einen Rest der Formel 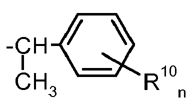 steht, worin
R¹⁰ für Halogen, Alkyl mit 1 bis 4 Kohlenstoffatomen oder Alkoxy mit 1 bis 4 Kohlenstoffatomen steht und
n für ganze Zahlen von 0 bis 3 steht.

2. Verfahren zur Herstellung von Isothiazolcarbonsäureamiden der Formel (I) gemäß Anspruch 1, **dadurch gekennzeichnet, dass** man
a) 3,4-Dichlor-isothiazol-5-carbonsäurechlorid der Formel 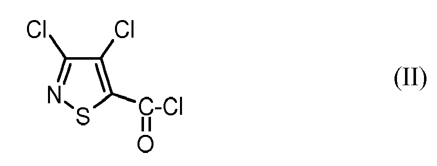 mit Aminen der Formel
H₂N-R (III)
in welcher
R die oben angegebenen Bedeutungen hat,
gegebenenfalls in Gegenwart eines Säurebindemittels und gegebenenfalls in Gegenwart eines Verdünnungsmittels umsetzt,
oder
b) 3,4-Dichlor-isothiazol-5-carbonsäureamid der Formel 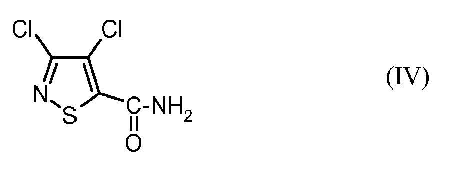 mit Hydroxyverbindungen der Formel
HO-CH₂-X (V)
in welcher
X für einen Rest der Formel 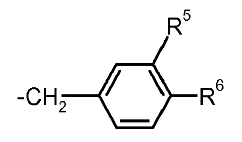 steht, worin
R⁵, R⁶ die oben angegebenen Bedeutungen haben,
in Gegenwart eines Verdünnungsmittels und in Gegenwart eines wasserabspaltenden Mittels umsetzt.

3. Mittel zum Schutz von Pflanzen gegen Befall durch unerwünschte Mikroorganismen und tierische Schädlinge, **gekennzeichnet durch** einen Gehalt an mindestens einem Isothiazolcarbonsäureamid der Formel (I) gemäß Anspruch 1 neben Streckmitteln und/oder oberflächenaktiven Stoffen.

4. Verwendung von Isothiazolcarbonsäureamiden der Formel (I) gemäß Anspruch 1 zum Schutz von Pflanzen gegen Befall durch unerwünschte Mikroorganismen und tierische Schädlinge.

5. Verfahren zum Schutz von Pflanzen gegen Befall durch unerwünschte Mikroorganismen und tierische Schädlinge, **dadurch gekennzeichnet, dass** man Isothiazolcarbonsäureamide der Formel (I) gemäß Anspruch 1 auf die Pflanzen und/oder deren Lebensraum ausbringt.

6. Verfahren zur Herstellung von Mitteln zum Schutz von Pflanzen gegen Befall durch unerwünschte Mikroorganismen und tierische Schädlinge, **dadurch gekennzeichnet, dass** man Isothiazolcarbonsäureamide der Formel (I) gemäß Anspruch 1 mit Streckmitteln und/oder oberflächenaktiven Stoffen vermischt.

7. Isothiazolcarbonsäureamid gemäß Anspruch 1, **gekennzeichnet durch** die Formel

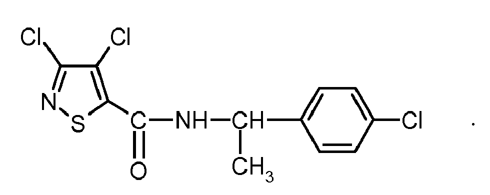

8. Isothiazolcarbonsäureamid gemäß Anspruch 1, **gekennzeichnet durch** die Formel
